# EUROPEAN PATENT APPLICATION

(11) **EP 2 315 047 A1**
(43) Date of publication of application: **27.04.2011**
(21) Application number: 10187086.3
(22) Date of filing: 11.10.2010
(51) Int. Cl.: G01S 7/52, A61B 8/00, G01S 15/89

(54) **Probe of ultrasonic diagnostic apparatus and control method thereof**

(30) Priority: 21.10.2009 KR 20090100055
(71) Applicant: Medison Co., Ltd., Kangwon-do 250-875 (KR)
(72) Inventor: Hyeon, Yong Cheol, Gyeonggi-do (KR)
(74) Representative: Schmid, Wolfgang

(57) **Abstract**

A probe (100) of an ultrasonic diagnostic apparatus (10) and a method of controlling the same are disclosed. The probe (100) includes transducer (110) movably disposed inside a housing (140) of the probe (100), a detector (130) detecting movement of the probe (100), and a drive unit (120) controlling operation of the transducer (110) in response to an output from the detector (130). Acceleration of the probe (100) is measured to adjust intensity of a holding current in order to suppress operation of the transducer (110) depending on the acceleration of the probe (100), so that, if there is no movement of the probe (100), a low holding current is output to increase a transmission current by suppressing heat generation, thereby improving image quality.

## Description

### BACKGROUND OF THE INVENTION

### 1. Field of the Invention

The present invention relates to a probe of an ultrasonic diagnostic apparatus and a control method thereof and, more particularly, to a probe of an ultrasonic diagnostic apparatus and a control method thereof that can measure acceleration of the probe and adjust intensity of holding current to suppress operation of a transducer depending on the acceleration of the probe.

### 2. Description of the Related Art

Generally, an ultrasonic diagnostic apparatus refers to a non-invasive apparatus that irradiates an ultrasound signal from a surface of a target towards a target internal organ beneath the surface of the target and obtains an image of a monolayer or blood flow in soft tissue from information in the reflected ultrasound signal (ultrasound echo-signal).

The ultrasonic diagnostic apparatus has been widely used for diagnosis of the heart, the abdomen, the urinary organs, and in obstetrics and gynecology due to various merits thereof such as small size, low price, real-time image display, and high stability through elimination of radiation exposure, as compared with other image diagnostic systems, such as X-ray diagnostic systems, computerized tomography scanners (CT scanners), magnetic resonance imagers (MRIs), nuclear medicine diagnostic apparatuses, and the like.

The ultrasonic diagnostic apparatus includes a probe which transmits an ultrasound signal to a target and receives an ultrasound echo-signal reflected therefrom to obtain an ultrasound image of the target.

The probe includes a transducer, a case with an open upper end, a cover coupled to the open upper end of the case to directly contact the surface of a target, and the like. The transducer includes a piezoelectric layer in which a piezoelectric material converts electrical signals into sound signals or vice versa while vibrating, a sound matching layer reducing a difference in sound impedance between the piezoelectric layer and the target to allow as much of the ultrasound waves generated from the piezoelectric layer as possible to be transferred to the target, a lens layer focusing the ultrasound waves, which travel in front of the piezoelectric layer, onto a predetermined point, and a backing layer blocking the ultrasound waves from traveling in a rearward direction of the piezoelectric layer to prevent image distortion.

In order to obtain a desired ultrasound image, the probe is moved on a body surface of the target or is rotated thereon while being brought into contact with the body surface of the target.

It should be noted that the above description is provided for understanding of the background of the invention and is not a description of a conventional technique well-known in the art.

In an ultrasonic diagnostic apparatus capable of providing a three-dimensional (3D) ultrasound image, a 3D probe is provided therein with a transducer which can move along an arcuate locus centered on a drive unit inside a housing of the probe to transmit an ultrasound echo-signal to a main body of the apparatus to generate a 3D ultrasound image of a target.

When the 3-D probe is used not in a 3-D mode but in a two-dimensional (2-D) mode, a constant intensity of holding current is output from the drive unit to suppress movement of the transducer in order to prevent a positional change of the transducer by impact from outside.

However, when the constant intensity of holding current is continuously output, the probe undergoes an increase in surface temperature due to heat generated during the operation in the 2-D mode, thereby restricting an increase in transmission voltage while increasing power consumption.

Further, when the intensity of the holding current is limited to a certain level or less due to heat generated from the drive unit, the position of the transducer is liable to be changed upon application of impact exceeding a certain degree.

### SUMMARY OF THE INVENTION

The present invention is conceived to solve the problems of the related art as described above, and an aspect of the invention is to provide a probe of an ultrasonic diagnostic apparatus and a control method thereof that can measure acceleration of the probe and adjust intensity of holding current to suppress operation of a transducer depending on the acceleration of the probe.

In accordance with one aspect of the invention, a probe of an ultrasonic diagnostic apparatus includes: a transducer movably disposed inside a housing of the probe; a detector detecting movement of the probe; and a drive unit controlling operation of the transducer in response to an output from the detector.

The drive unit may include an operation controller outputting an operating current to swing the transducer, or receiving the output from the detector and outputting a holding current to suppress the operation of the transducer; and a motor receiving the operating current or the holding current from the operation controller and operating the transducer.

The detector may be disposed near the transducer.

The detector may include an acceleration sensor.

In accordance with another aspect of the invention, a method of controlling a probe of an ultrasonic diagnostic apparatus includes: determining whether the probe is in a three-dimensional mode; outputting an operating current to swing a transducer if the probe is in the three-dimensional mode, or determining whether there is movement of the probe in response to an output from a detector detecting the movement of the probe if the probe is not in the three-dimensional mode; and outputting a holding current to suppress operation of the transducer depending on whether there is movement of the probe.

The outputting a holding current may include outputting a low holding current if there is no movement of the probe and outputting a high holding current obtained by adjusting intensity of the holding current depending on acceleration of the probe if there is movement of the probe.

The method may further include outputting an operating current to return the transducer to an original position, if it is determined that the probe moves at a predetermined acceleration or more.

### BRIEF DESCRIPTION OF THE DRAWINGS

The above and other aspects, features and advantages of the invention will become apparent from the following description in conjunction with the accompanying drawings, in which:
- Fig. 1: is a perspective view of an ultrasonic diagnostic apparatus including a probe in accordance with one embodiment of the present invention;
- Fig. 2: is a block diagram of the probe of Fig. 1;
- Fig. 3: is a schematic cross-sectional view of the probe of Fig. 1;
- Fig. 4: is a flowchart of a method of controlling a probe of an ultrasonic diagnostic apparatus in accordance with one embodiment of the present invention; and
- Fig. 5: is a graph depicting a holding current adjusted by the method in accordance with the embodiment of the present invention.

### DETAILED DESCRIPTION OF THE EMBODIMENT

Exemplary embodiments of the invention will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the invention into account and can be changed according to the custom or intention of users or operators. Therefore, definition of the terms should be made according to overall disclosures set forth herein.

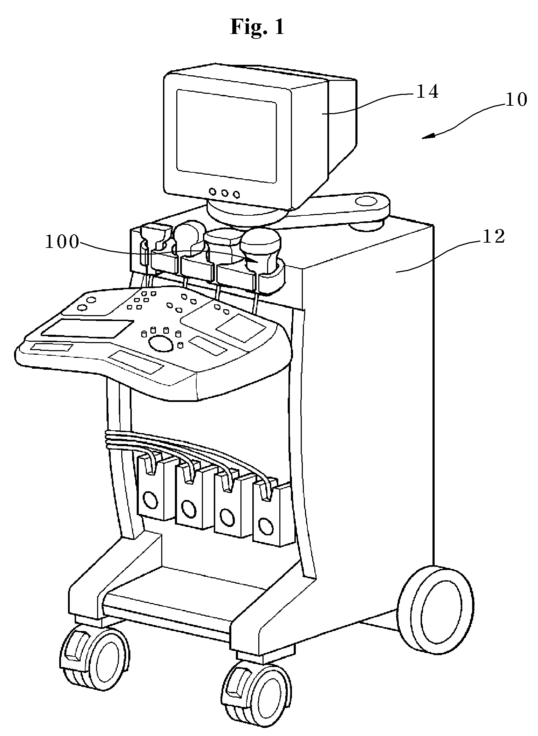

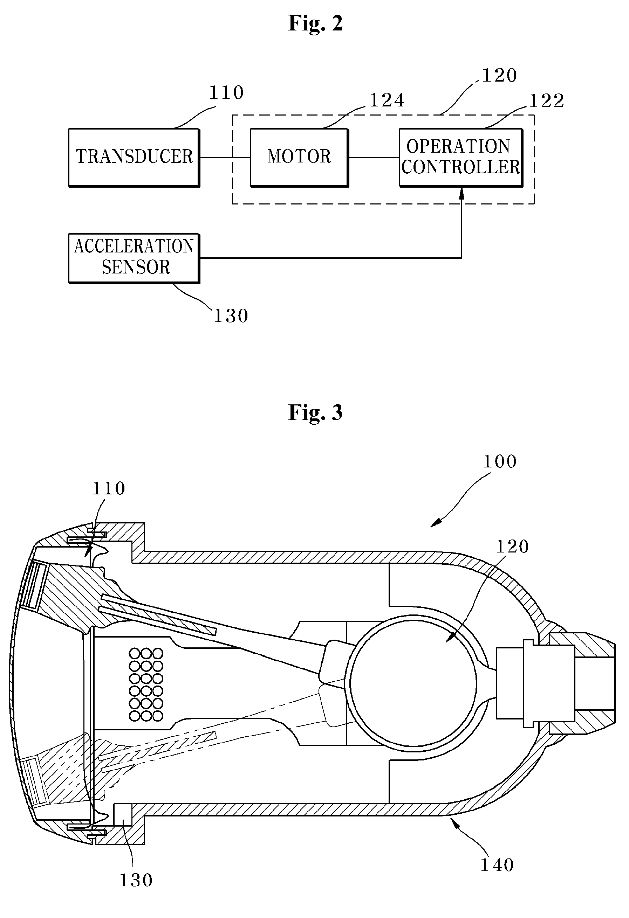

Fig. 1 is a perspective view of an ultrasonic diagnostic apparatus including a probe in accordance with one embodiment of the present invention; Fig. 2 is a block diagram of the probe of Fig. 1; and Fig. 3 is a schematic cross-sectional view of the probe of Fig. 1.

Referring to Fig. 1, a probe 100 according to one embodiment is provided to an ultrasonic diagnostic apparatus 10. The ultrasonic diagnostic apparatus 10 includes a main body 12 for controlling or manipulating the apparatus 10, and a display unit 14 for displaying ultrasound images generated by the main body 12.

The probe 100 transmits an ultrasound signal to a target and receives and transmits a reflected ultrasound echo-signal to the main body 12.

Referring to Figs. 2 and 3, the probe 100 includes a transducer 110, a drive unit 120, and an acceleration sensor 130 acting as a detector for measuring movement of the probe 100.

The transducer 110 includes a piezoelectric layer (not shown) in which a piezoelectric material converts electrical signals into sound signals or vice versa while vibrating, a sound matching layer (not shown) reducing a difference in sound impedance between the piezoelectric layer and a target to allow as much of the ultrasound signals generated from the piezoelectric layer as possible to be transferred to the target, and a lens layer (not shown) focusing the ultrasound signals, which travel in front of the piezoelectric layer, onto a predetermined point.

The transducer 110 is movably disposed inside a housing 140 of the probe 100.

When there is movement of the probe 100, the acceleration sensor 130 measures the movement of the probe 100 and outputs acceleration of the probe 100 to the drive unit 120.

The acceleration sensor 130 is located near the transducer 110 inside the housing 140 of the probe 100 to sense movement of the transducer 110.

The position of the acceleration sensor 130 is not limited thereto, and the acceleration sensor 130 may be located at any position so long as it can sense the movement of the transducer 110 by measuring the acceleration of the probe 100.

The drive unit 120 is disposed inside the housing 140 and operates the transducer 110. While the transducer 110 moves along an arcuate locus centered on the drive unit 120, the probe 100 transmits an ultrasound echo-signal to the main body 12 of the diagnostic apparatus 10 to realize a 3D ultrasound image.

In response to operation control of the diagnostic apparatus 10, the drive unit 120 allows the transducer 110 to swing in a 3D mode and suppresses operation of the transducer 110 in a 2D (two dimensional) mode depending on the acceleration output from the acceleration sensor 130.

The drive unit 120 includes an operation controller 122 and a motor 124.

The operation controller 122 outputs an operating current to the motor 124 to allow the transducer 110 to swing, or receives the acceleration output from the acceleration sensor 130 and outputs a holding current to the motor 124 to suppress the operation of the transducer 110. The motor 124 operates the transducer 110 according to the operating current or the holding current output from the operation controller 122.

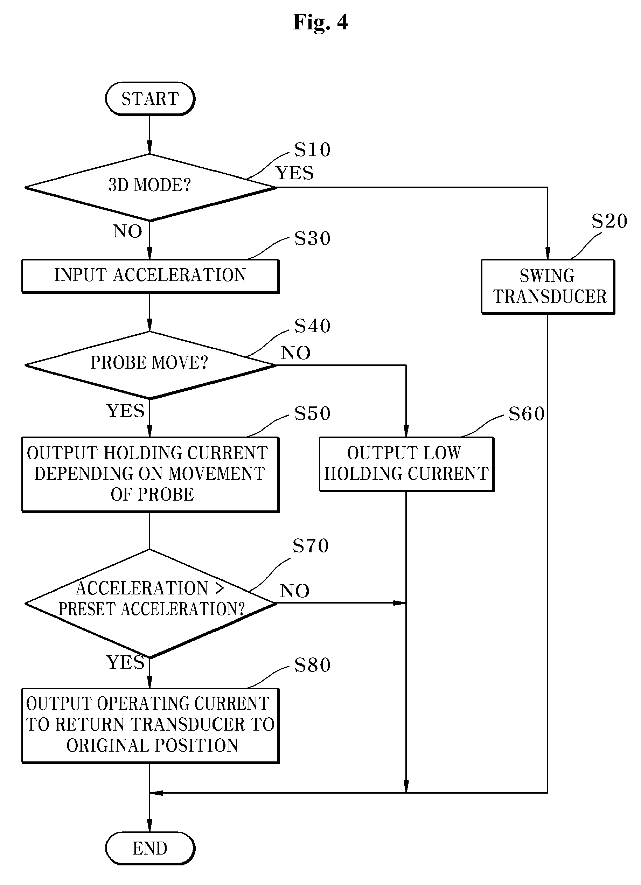

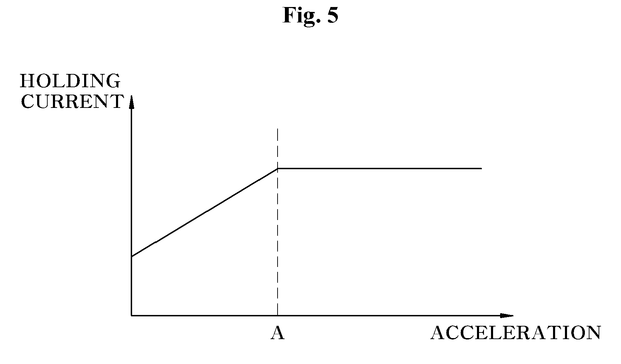

Fig. 4 is a flowchart of a method of controlling a probe of an ultrasonic diagnostic apparatus in accordance with one embodiment of the present invention, and Fig. 5 is a graph depicting a holding current adjusted by the method in accordance with the embodiment of the present invention.

Referring to Figs. 1 to 5, in the method according to this embodiment, first, the operation controller 122 determines whether a diagnostic mode of the ultrasonic diagnostic apparatus 10 is a three-dimensional mode, in S10.

If the diagnostic apparatus is in the 3D mode, the operation controller outputs an operating current to the motor 124 to swing the transducer 110, in S20.

If the diagnostic apparatus 10 is not in the 3D mode but in a 2D mode, the operation controller receives an output from the acceleration sensor 130 detecting movement of the probe 100 in order to adjust and output a holding current for suppressing operation of the transducer 110, in S30.

In response to the output from the acceleration sensor 130, the operation controller determines whether there is movement of the probe 100, in S40.

If there is no movement of the probe 100, the operation controller outputs a low holding current to the motor 124 since the operation of the transducer 110 can be suppressed with a low force, in S60.

As such, if there is no movement of the probe 100, a low holding current is output to the motor 124 so that the surface temperature of the probe 100 is prevented from being increased by heat from the motor 124.

If the movement of the probe 100 is detected, the operation controller adjusts and outputs a holding current depending on the acceleration measured by the acceleration sensor 130 to suppress the operation of the transducer 110, in S50.

Here, when outputting the holding current, the operation controller adjusts the holding current in proportion to acceleration of the probe until the acceleration of the probe reaches a preset acceleration "A", as shown in Fig. 5.

Then, it is determined whether the acceleration of the probe measured by the acceleration sensor 130 exceeds the preset acceleration "A", in S70.

Here, when there is strong impact from outside or when there is an abrupt movement of the probe 100, the acceleration of the probe exceeds the preset acceleration "A". In this case, the operation controller outputs a high holding current to suppress the operation of the transducer 110 and an operation current to return the transducer 110 to its original position, in S80.

As such, according to the embodiment of the invention, acceleration of the probe may be measured to control intensity of a holding current, which is used for suppressing operation of the transducer, depending on the acceleration of the probe, so that, if there is no movement of the probe, a low holding current is output to increase a transmission current by suppressing heat generation, thereby improving image quality.

Further, according to the embodiment, the probe may be operated at a low holding current, so that a battery used in an ultrasonic diagnostic apparatus has a long lifespan.

Further, according to the embodiment, when strong impact is applied from outside to the transducer or when the probe is abruptly moved, a high holding current may be output to prevent movement of the transducer.

Further, according to the embodiment, when the transducer is moved by a certain level of impact or more, the transducer may be returned to an original position.

Although some embodiments have been provided to illustrate the invention in conjunction with the drawings, it will be apparent to those skilled in the art that the embodiments are given by way of illustration only, and that various modifications and equivalent embodiments can be made without departing from the spirit and scope of the invention. The scope of the invention should be limited only by the accompanying claims.

## Claims

1. A probe (100) of an ultrasonic diagnostic apparatus (10), **characterized by** comprising:
a transducer (110) movably disposed inside a housing (140) of the probe (100);
a detector (130) detecting movement of the probe (100); and
a drive unit (120) controlling operation of the transducer (110) in response to an output from the detector (130).

2. The probe (100) according to claim 1, wherein the drive unit (120) comprises:
an operation controller (122) outputting an operating current to swing the transducer (100), or receiving the output from the detector (130) and outputting a holding current to suppress the operation of the transducer (110); and
a motor (124) receiving the operating current or the holding current from the operation controller (122) and operating the transducer (110).

3. The probe (100) according to claim 1, wherein the detector (130) is disposed near the transducer (110).

4. The probe (100) according to any one of claims 1 to 3, wherein the detector (130) comprises an acceleration sensor.

5. A method of controlling a probe of an ultrasonic diagnostic apparatus, comprising:
determining whether the ultrasonic diagnostic apparatus is in a three-dimensional mode;
outputting an operating current to swing a transducer if the ultrasonic diagnostic apparatus is in the three-dimensional mode, or determining whether there is movement of the probe in response to an output from a detector detecting the movement of the probe if the ultrasonic diagnostic apparatus is not in the three-dimensional mode; and
outputting a holding current to suppress operation of the transducer depending on whether there is movement of the probe.

6. The method according to claim 5, wherein the outputting a holding current comprises :
outputting a low holding current if there is no movement of the probe, and
outputting a high holding current obtained by adjusting intensity of the holding current depending on acceleration of the probe if there is the movement of the probe.

7. The method according to claim 5, further comprising:
outputting an operating current to return the transducer to an initial position, if it is determined that the probe moves at a predetermined acceleration or more.
